# EUROPÄISCHE PATENTSCHRIFT

(11) **EP 2 314 276 B1**
(45) Veröffentlichungstag und Bekanntmachung des Hinweises auf die Patenterteilung: **17.07.2013**
(21) Anmeldenummer: 11154398.9
(22) Anmeldetag: 18.04.2007
(51) Int. Cl.: A61K 8/02, A61K 8/04, A61K 8/06, A61K 8/44, A61Q 19/00

(54) **Dispersionen, enthaltend Acylglutamate**
Dispersions containing acylglutamate
Dispersions comprenant des acylglutamates

(30) Priorität: 27.04.2006 EP 06008764
(43) Veröffentlichungstag der Anmeldung: 27.04.2011
(62) Teilanmeldung aus: 07724329.3
(73) Patentinhaber: Cognis IP Management GmbH, 40589 Düsseldorf (DE)
(72) Erfinder: Kawa, Rolf, 40789, Monheim (DE); Eskuchen, Rainer, 40764, Langenfeld (DE); Goget, Caroline, 75018, Paris (FR)

(56) Entgegenhaltungen:
- EP-A2- 1 502 644
- US-A1- 2002 034 480

## Beschreibung

### Gebiet der Erfindung

Die Erfindung befindet sich auf dem Gebiet der Dispersionen und betrifft feinteilige, besonders lagerstabile Dispersionen, enthaltend Acylglutamate.

### Stand der Technik

Der Stand der Technik beschreibt eine Vielzahl von kosmetischen Emulsionen mit einer Vielzahl verschiedenster Emulgatoren. So beschreibt z.B. EP 1 502 644 A2 Emulgatorkombinationen, welche frei sind von ethoxylierten Emulgatoren und Alkyloligoglucoside, Polyolpolyhydroxystearate und Acylglutamate enthalten und sich zur Herstellung von Nanoemulsionen eignen. Die in EP 1 502 644 beschriebenen Emulsionen enthalten alle Gesamtemulgatormengen von über 3 Gew.-%. US 2002/034480 offenbart ähnliche Emulsionen.

Aufgabe der vorliegenden Erfindung hat darin bestanden lagerstabilen Dispersionen mit möglichst geringem Anteil an Emulgator bereitzustellen. Von besonderem Interesse war die Phasenstabilität, insbesondere bei Lagerung bei erhöhten Temperaturen, und gleichzeitig hohe sensorische Leistung (Weichheit, Pflege) der Dispersion. Gewünscht war weiterhin, dass sich große Mengen an lipophiler Phase im Verhältnis zur Gesamtemulgatormenge zu einer stabilen Dispersion verarbeiten lassen. Aufgabe der Erfindung war weiterhin, dass sich die Dispersion verdünnen lässt unter Erhalt der Teilchengrößenverteilung der Dispersion.

Überraschenderweise wurde festgestellt, dass Dispersionen gemäß Anspruch 1 diese Aufgabe lösen. Die erfindungsgemäßen Dispersionen sind lagerstabil, weisen hervorragende sensorische Eigenschaften bezüglich Pflege und Weichheit auf, und ermöglichen die Einarbeitung großer Mengen an lipophiler Phase. Die erfindungsgemäßen Dispersionen zeigen insbesondere bei Lagerung bei erhöhten Temperaturen über einen Zeitraum von mehreren Wochen keine Phasentrennung. Des Weiteren können die erfindungsgemäßen Dispersionen verdünnt werden ohne dass sich die Teilchengrößenverteilung der Dispersion ändert.

### Beschreibung der Erfindung

Gegenstand der Erfindung sind Dispersionen gemäß Anspruch 1

### Dispersionen

Als Dispersion wird ein System (disperses System) aus mehreren Phasen bezeichnet, von denen eine kontinuierlich (Dispersionsmittel) und mindestens eine weitere fein verteilt ist (dispergierte Phase, Dispergens). Beispiele für Dispersionen sind: Emulsionen (Dispergiermittel und Dispergens: flüssige, ineinander unlösliche Phasen), Aerosole [Dispersionsmittel gasförmig, Dispergens flüssig (= Nebel) oder fest (=Rauch, Staube)], Suspensionen (Dispersionsmittel flüssig, Dispergens fest).

Alle Gew.% Angaben beziehen sich- sofern nicht anders angegeben- auf das Gesamtgewicht der Dispersion.

### Acylglutamate.

Acylglutamate sind bekannte Emulgatoren, die der Formel **(I)** folgen, 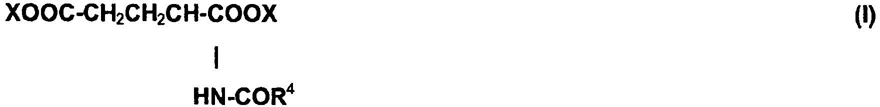 in der R⁴CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht. Ihre Herstellung erfolgt beispielsweise durch Schotten-Baumann-Acylierung von Glutaminsäure mit Fettsäuren, Fettsäureestern oder -chloriden. Verkaufsprodukte sind beispielsweise von der Hoechst AG, Frankfurt/DE oder der Ajinomoto Co. Inc., Tokio/JP erhältlich. Eine Übersicht zu Herstellung und Eigenschaften der Acylglutamate findet sich von M. Takehara et al. in J. Am. Oil Chem. Soc. 49 (1972) 143. Typische Beispiele für geeignete Acylglutamate, die im Sinne der Erfindung in Betracht kommen, sind diejenigen Acylglutamate, die sich von Fettsäuren mit 6 bis 22 und vorzugsweise 12 bis 18 Kohlenstoffatomen ableiten. Besonders bevorzugt sind Acylglutamate, die sich von Fettsäuren mit 16 bis 18 Kohlenstoffatomen ableiten, insbesondere von Fettsäuren mit 18 Kohlenstoffatomen (Stearoylglutamate). Besonders bevorzugt sind Kokosfettsäureglutamate, beispielsweise der C_{12/14}- bzw. C_{12/18}-Kokosfettsäure. Es werden insbesondere die Mono- oder Dialkalimetallsalze des Acylglutamats eingesetzt. Besonders bevorzugt sind Natriumsalze des Stearoylglutamates (INCI: Sodium Stearoyl Glutamate), wie sie beispielsweise unter dem Handelsnamen Eumulgin ®SG (Cognis Deutschland GmbH & Co. KG), oder Amisoft HS-11 P (Ajinomoto, USA) verkauft werden.

Die im Rahmen der Erfindung verwendeten Begriffe Acylglutamat bzw. Acylglutamate umfassen sowohl einzelne Acylglutamate als auch beliebige Mischungen verschiedener Acylglutamate.

### Emulgator

Als Emulgatoren im Sinne der Erfindung werden solche Stoffe verstanden, die einen HLB-wert von größer gleich 10, vorzugsweise größer gleich 12 aufweisen.

Die erfindungsgemäßen Dispersionen enthalten weniger als 3 Gew.-% Emulgator bezogen auf die Gesamtmenge der Dispersion. In einer bevorzugten Ausführungsform der Erfindung enthält der Emulgator mehr als 30 Gew.-%, vorzugsweise mehr als 50 Gew.-% Acylglutamate. In einer bevorzugten Ausführungsform enthält der Emulgator mehr als 60, insbesondere mehr als 70, vorzugsweise mehr als 80 Gew.%, vorzugsweise mehr als 90 Gew.-% Acylglutamate. In einer Ausführungsform der Erfindung besteht der Emulgator zu 100 % aus Acylglutamaten.

Sofern die Dispersionen Phospholipide enthalten, werden diese nicht zu den Emulgatoren gerechnet.

Ein hoher Anteil an Acylglutamaten am Emulgator hat sich besonders vorteilhaft für die Lagerstabilität der Dispersionen erwiesen.

Neben den Acylglutamaten können die erfindungsgemäßen Dispersionen übliche weitere Emulgatoren oder Tenside enthalten. Die Auswahl richtet sich zweckmäßig nach der beabsichtigten Verwendung der Dispersion und insbesondere nach der Art der lipophilen Phase, die emulgiert werden soll. Als weitere Emulgatoren ist bevorzugt mindestens ein nicht-ionischer Emulgator enthalten. Als nicht ionische Emulgatoren seien exemplarisch genannt Alkyloligoglycoside, Polyolpolyhydroxystearate sowie Sorbitanmono- oder -diester.

Als weitere Emulgatoren ist bevorzugt mindestens ein anionischer Emulgator enthalten.

In einer bevorzugten Ausführungsform der Erfindung enthalten die Dispersionen weniger als 2,5 Gew.-%, insbesondere weniger als 2 Gew.-%, bevorzugt weniger als 1 Gew.-% an Emulgator.

In einer bevorzugten Ausführungsform der Erfindung enthalten die Dispersionen weniger als 2,5 Gew.-%, insbesondere weniger als 2 Gew.-%, bevorzugt weniger als 1 Gew.-% an Emulgator, wobei der Emulgator jeweils mehr als 30 Gew.-%, vorzugsweise mehr als 50 Gew.-% Acylglutamate, bevorzugt mehr als 60, insbesondere mehr als 70, vorzugsweise mehr als 80 Gew.%, vorzugsweise mehr als 90 Gew.-% Acylglutamate enthält.

In einer bevorzugten Ausführungsform der Erfindung, enthält der Emulgator weniger als 10 Gew.-%, vorzugsweise weniger als 5 Gew.-% ethoxylierte Emulgatoren, insbesondere weniger als 2 Gew.% ethoxylierte Emulgatoren. In einer bevorzugten Ausführungsform der Erfindung enthalten die Dispersionen keine ethoxylierten Emulgatoren.

Eine Ausführungsform der Erfindung umfasst Dispersionen, dadurch gekennzeichnet, dass der Emulgator in einer Menge von kleiner gleich 3 Gew.-% bezogen auf das Gesamtgewicht der Dispersion enthalten ist und dass der Emulgator mindestens ein Acylglutamat enthält und dass der Emulgator weniger als 10 Gew.-% ethoxylierte Emulgatoren enthält.

Eine Ausführungsform der Erfindung umfasst Dispersionen, dadurch gekennzeichnet, dass der Emulgator in einer Menge von kleiner gleich 3 Gew.-% bezogen auf das Gesamtgewicht der Emulsion enthalten ist und dass der Emulgator mehr als 30 Gew.-%, vorzugsweise mehr als 50 Gew.-% Acylglutamate enthält und dass der Emulgator weniger als 10 Gew.-% ethoxylierte Emulgatoren enthält.

### Weitere Emulgatoren

Als übliche weitere Emulgatoren seinen die folgenden Stoffe genannt, sofern Sie einen HLB-wert von größer gleich 10, vorzugsweise größer gleich 12 aufweisen.

Cetyl Dimethicone Copolyol (z. B. Abil EM-90), Polyglyceryl-2 Dipolyhydroxystearate (z. B. Dehymuls PGPH), Polyglycerin-3-Diisostearate (z. B. Lameform TGI), Polyglyceryl-4 Isostearate (z. B. Isolan GI 34), Polyglyceryl-4 Düsostearate/Polyhydroxystearate/Sebacate (Isolan GPS), Polyglyceryl-3 Oleate, Düsostearoyl Polyglyceryl-3 Diisostearate (z. B. Isolan PDI), Polyglyceryl-3 Methylglucose Distearate (z. B. Tego Care 450), Polyglyceryl-3 Beeswax (z. B. Cera Bellina), Polyglyceryl-4 Caprate (z. B. Polyglycerol Caprate T2010/90), Polyglyceryl-3 Cetyl Ether (z. B. Chimexane NL), Polyglyceryl-3 Distearate (z. B. Cremophor GS 32) und Polyglyceryl Polyricinoleate (z. B. Admul WOL 1403), Glyceryl Oleate (z. B. Monomuls 90-O 18), Alkyl Glucoside (z. B. Plantacare 1200, Emulgade PL 68/50, Montanov 68, Tego Care CG 90, Tego Glucosid L 55), Methyl Glucose Isostearate (z. B. Tego Care IS), Methyl Glucose Sesquistearate (Tego Care PS), Sodium Cocoyl Hydrolyzed Wheat Protein (z. B. Gluadin WK), Alkalisalze von Alkyl Phosphaten (z. B. Amphisol K, Crodafos CKP), Sodium Alkylsulfate (z. B. Lanette E), Sucrose Ester (z. B. Crodesta F-10, F-20, F-50, F-70, F-110, F-160, SL-40, Emulgade Sucro), ethoxylierte und/oder propoxylierte Fettalkohole Fettsäuren, Rizinusöle bzw. hydrierte Rizinusöle (z. B. Eumulgin B1, B2, B3, L, HRE 40, HRE 60, RO 40 Cremophor HRE 40, HRE 60, L, WO 7, Dehymuls HRE 7, Arlacel 989), PEG-30 Dipolyhydroxystearate (Dehymuls LE, Arlacel P 135), Sorbitan Ester, Sorbitan Ester ethoxyliert und/oder propoxyliert, sowie deren Gemische.

Als weitere Emulgatoren kommen beispielsweise nicht-ionische Emulgatoren aus mindestens einer der folgenden Gruppen in Frage:
- Alkyl- und/oder Alkenyloligoglykoside mit 8 bis 22 Kohlenstoffatomen im Alk(en)ylrest
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

### Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosid-restes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### Phospholipide

In einer bevorzugten Ausführungsform der Erfindung enthält die Dispersion weiterhin mindestens ein Phospholipid.

Kommerzielle erhältliche "Phospholipide" stellen üblicherweise komplexe Gemische dar, die je nach Herkunft und Gewinnungsmethode, zusätzlich Sterole, Öle etc. enthalten können.

Phospholipide sind komplexe Lipide, in denen eine der primären Hydroxylgruppen des Glycerins mit Phosphorsäure verestert ist, welche wiederum verestert ist. Die 2 übrigen Hydroxylgruppen des Glycerins sind mit langkettigen, gesättigten oder ungesättigten Fettsäuren verestert.

Phospholipide sind Phosphorsäuredi-, oder -monoester, die wegen ihrer fettähnlichen Löslichkeitseigenschaften aufgrund der lipophilen und hydrophilen Komponenten zu den Lipiden gerechnet werden und im Organismus als Membranlipide am Aufbau von Schichten-Strukturen, den Membranen, beteiligt sind. Phosphatidsäuren sind Glycerinderivate, die in 1-sn- und 2-Stellung mit Fettsäuren (1-sn-Position: meist gesättigt, 2-Position: meist ein- oder mehrfach ungesättigt), an Atom 3-sn dagegen mit Phosphorsäure verestert sind und durch die allgemeine Strukturformel 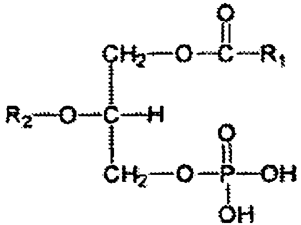 gekennzeichnet sind.

In den in menschlichem oder tierischem Gewebe vorkommenden Phosphatidsäuren ist der Phosphatrest meist verestert mit Aminoalkoholen wie Cholin (Lecithin = 3-sn-**Phosphatidylcholin**) oder 2-Aminoethanol (Ethanolamin) bzw. L-Serin (Kephalin = 3-sn-Phosphatidylethanolamin bzw. sn-Phosphatidyl-L-serin), mit myo-Inosit zu den in Geweben häufigen Phosphoinositiden [1-(3-sn-Phosphatidyl)-D-myo-inositen], mit Glycerin zu Phosphatidylglycerinen.

**Lecithine** sind durch die allgemeine Strukturformel gekennzeichnet, 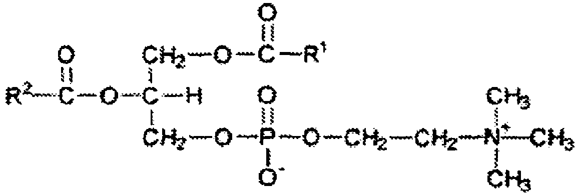 wobei R¹ und R² typischerweise unverzweigte aliphatische Reste mit 15 oder 17 Kohlenstoffatomen und bis zu 4 cis Doppelbindungen darstellen.

**Cardiolipine** (1,3-Bisphosphatidylglycerine) sind Phospholipide aus zwei über Glycerin verknüpften Phosphatidsäuren. Lysophospholipide werden erhalten, wenn aus Phospholipiden ein Acylrest durch Phospholipase A abgespalten wird (z.B. Lysolecithine).

**Lysophospholipide** sind gekennzeichnet durch die allgemeine Strukturformel

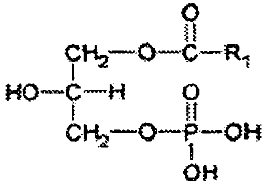

Lysolecithine beispielsweise sind gekennzeichnet durch die allgemeine Strukturformel 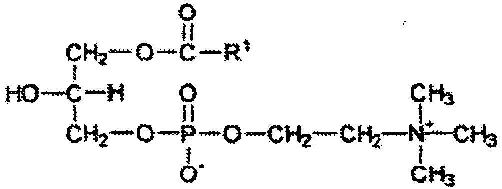 wobei R¹ und R² typischerweise unverzweigte aliphatische Reste mit 15 oder 17 Kohlenstoffatomen und bis zu 4 cis Doppelbindungen darstellen.

Zu den Phospholipide werden auch Plasmalogene gezählt, in denen statt einer Fettsäure in 1-Stellung ein Aldehyd (in Form eines Enolethers) gebunden ist; die den Phosphatidylcholinen entsprechenden O-1-sn-Alkenyl-Verbindungen z. B. heißen Phosphatidalcholine.

Den Phosphosphingolipiden liegt als Grundstruktur das Sphingosin oder auch das Phytosphingosin zugrunde, welche sich durch folgende Strukturformeln auszeichnen:

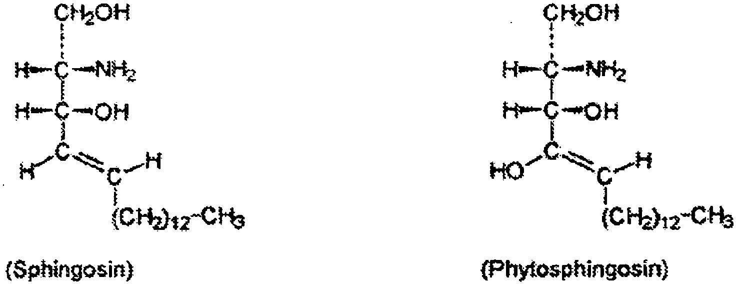

Abwandlungen von Sphingolipiden zeichnen sich beispielsweise aus durch die allgemeine Grundstruktur 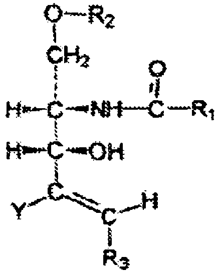 bei welcher R₁ und R₃ unabhängig voneinander gesättigte oder ungesättigte, verzweigte oder unverzweigte Alkylreste von 1 bis 28 Kohlenstoffatomen darstellen, R₂ gewählt wird aus der Gruppe: Wasserstoffatom, gesättigte oder ungesättigte, verzweigte oder unverzweigte Alkylreste von 1 bis 28 Kohlenstoffatomen, Zuckerreste, mit organischen Resten veresterte oder unveresterte Phosphatgruppen, mit organischen Resten veresterte oder unveresterte Sulfatgruppen und Y entweder ein Wasserstoffatom, eine Hydroxygruppe oder einen anderen hetero-funktionellen Rest darstellt.

### Sphingophospholipide:

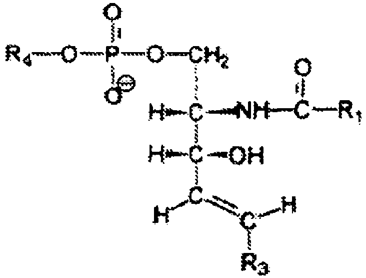

R₁ und R₃ stellen Alkylreste dar, R₄ stellt einen Organylrest dar. Sphingomyeline sind organylphosphorylierte Sphingolipide des Typs

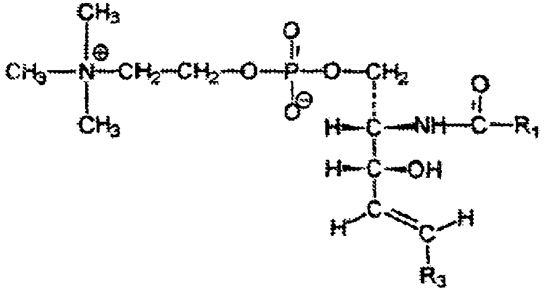

Im Rahmen dieser Erfindung werden unter dem Begriff Phospholipide auch Phosphosphingolipide und Sphingophospholipide umfasst.

In einer bevorzugten Ausführungsform der Erfindung werden Phospholipde pflanzlichen Ursprungs eingesetzt. Diese können beispielsweise aus Soja oder anderen pflanzlichen Samenzellen gewonnen werden.

Vorteilhaft zu verwendende Phospholipide werden gewählt aus Phospholipiden, welche entölt und/oder fraktioniert und/oder sprühgetrocknet und/oder acetyliert und/oder hydrolysiert und/oder hydriert wurden.

Vorteilhaft zu verwendende Phospholipide werden gewählt aus Phospholipiden, welche Phosphatidylcholin angereichert und/oder Phosphatidylinositol angereichert sind.

Erfindungsgemäß vorteilhaft zu verwendende Phospholipide sind beispielsweise käuflich zu erwerben unter den Handelsbezeichnungen Leciprime 1800 IP (Fa. Cargill), Phosal 50 SA+ (Fa. Phospholipid), Soluthin MD (Fa. Phospholipid), Lipoid SL 80-3 (Fa. Lipoid). Die Menge der Phospholipide (eine oder mehrere Verbindungen) in den Emulsionen beträgt vorzugsweise 0,01 bis 10 Gew.-%, besonders bevorzugt 0,1 - 8 Gew.-%, insbesondere 0,5 - 5%, insbesondere 2 bis 4 Gew.-%, bezogen auf das Gesamtgewicht der Emulsion.

### Dispersionen

Eine Kenngröße, um Dispersionen bzw. die Größenverteilung der dispergierten Partikel zu beschreiben ist der Sauter Durchmesser. Der Sauter Durchmesser ist definiert als der Durchmesser eines Tropfens, mit dem gleichen Verhältnis von Tropfenvolumen zu Tropfenoberfläche, wie dieses als Mittelwert in der gesamten Dispersion vorliegt.

Der Sauter Durchmesser ist mathematisch definiert als D[3,2]: $D \left[3, 2\right] = {\overline{d}}_{m} = \frac{{\sum }_{{s}_{i}} {d}_{i}}{S} = \frac{{\sum }_{{n}_{i}} ⁢ {d}_{i}^{3}}{{\sum }_{{n}_{i}} ⁢ {d}_{i}^{2}}$

Worin *sᵢ* is the die Gesamtoberfläche, *nᵢ* π *d*²*ᵢ*, der Partikel innerhalb der Gruppe *i* ist und *S* die Gesamtoberfläche der Gesamtpopulation ist.

In einer bevorzugten Ausführungsform der Erfindung beträgt der Sauter Durchmesser d_{3,2} der Dispersion kleiner gleich 400 nm, insbesondere kleiner gleich 200 nm.

Eine Kenngröße, um Dispersionen (insbesondere Emulsionen) bzw. die Größenverteilung der dispergierten Partikel zu beschreiben ist die Breite der Tröpfchengrößenverteilung. Die Breite der Tröpfchengrößenverteilung kann durch den so genannten BTV Wert beschreiben werden: $BTV = \frac{{d}_{3 , 90} - {d}_{3 , 10}}{{d}_{3 , 50}}$

| | |
|---|---|
| d | Tropfendurchmesser |
| d_{3,90} | 90% des Volumens der dispersen Phase wird von Tropfen mit d <= d_{3,90} gebildet |
| d_{3,50} | 50% des Volumens der dispersen Phase wird von Tropfen mit d <= d_{3,50} gebildet |
| d_{3,10} | 10% des Volumens der dispersen Phase wird von Tropfen mit d <= d_{3,10} gebildet. |

Je kleiner der BTV Wert, desto enger ist die Tröpfchengrößenverteilung.
Bevorzugt weisen die erfindungsgemäßen Dispersionen einen BTV Wert von kleiner gleich 2, insbesondere kleiner gleich 1 auf.

### Lipophile Phase

Die erfindungsgemäßen Dispersionen enthalten eine lipophile Phase. Die lipophile Phase kann Ölkomponenten, Fette, Wachse sowie beliebige Mischungen daraus enthalten.

In einer bevorzugten Ausführungsform der Erfindung enthält die Dispersion 1 bis 80, vorzugsweise 10 bis 80, insbesondere 20 bis 70, bevorzugt 30 bis 60 Gew.-% lipophile Phase.

Die erfindungsgemäßen Dispersionen liegen je nach Wahl der lipophilen Phase als Suspensionen und/oder als Emulsionen vor. Dies kann durch Kombination verschiedener Fette, Öle und Wachse erfolgen oder indem die lipophile Phase ausschließlich aus einem ÖI besteht.

In einer bevorzugten Ausführungsform der Erfindung enthält die lipophile Phase nicht mehr als 25 Gew.-%, vorzugsweise nicht mehr als 20 Gew.-% (bezogen auf die gesamte lipophile Phase) lipophile Phase mit einem Polaritätsindex von größer gleich 35 mN/m und/oder einem Polaritätsindex von kleiner gleich 7 mN/m. Der Polaritätsindex einer lipophilen Phase kann mit einem Ringtensiometer (z.B: Krüss K 10 bestimmt werden, welches die Grenzflächenenergie misst, welche die Grenzflächenspannung in mN/M ist. Der untere Wert ist 5mN/m. Diese Methode eignet sich für Flüssigkeiten mit geringer Viskosität, sofern eine Grenzfläche vorhanden ist, d.h. die Flüssigkeiten nicht mischbar sind. Die Polarität der lipophilen Phase wird gegen Wasser bestimmt. Eine Methode um die Grenzflächenspannung zu messen ist beschrieben in ASTM Methode D971-99a (reapproved 2004).

Die Polarität verschiedener lipophiler Phasen ist beispielsweise in DE 1020004003436 A1 auf Seiten 8 bis 11 beschrieben, auf welche hier ausdrücklich Bezug genommen wird.

In einer Ausführungsform der Erfindung enthält die lipophile Phase mindestens ein ÖI.

Unter dem Begriff "Öle" (synonym verwendet: Ölkomponente) werden wasserunlösliche, bei 30°C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz.

Als Ölkomponenten sind beispielsweise die nachstehend genannten Verbindungsklassen geeignet, soweit diese bei 30°C flüssig sind. So z.B. Guerbetalkohole auf Basis von Fettalkoholen mit 6 bis 18, vorzugsweise 8 bis 10 Kohlenstoffatomen (z. B. Eutanol^{®} G), Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₃-C₃₈-Alkylhydroxycarbonsäuren mit linearen oder verzweigten C₆-C₂₂-Fettalkoholen - insbesondere Dioctylmalat -, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1,3-Dialkylcyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen (Hydagen^{®} HSP, Sovermol^{®} 750, Sovermol^{®} 1102), Siliconöle (Cyclomethicone, Siliciummethicontypen u.a. und/oder aliphatische bzw. naphthenische Kohlenwasserstoffe, wie z.B. wie Mineralöl, Vaseline, Petrolatum, Squalan, Squalen oder Dialkylcyclohexane in Betracht.

Als **Siliconöle** geeignet sind neben Dimethylpolysiloxanen, Methylphenylpolysiloxanen und cyclischen Siliconen sind amino-, fettsäure-, alkohol-, polyether-, epoxy-, fluor-, glykosid- und/oder al-kylmodifizierte Siliconverbindungen geeignet, die bei Raumtemperatur sowohl flüssig als auch harzförmig vor-liegen können. Weiterhin geeignet sind Simethicone, bei denen es sich um Mischungen aus Dimethiconen mit einer durchschnittlichen Kettenlänge von 200 bis 300 Dimethylsiloxan-Einheiten und Siliciumdioxid oder hydrierten Silicaten handelt.

Als Ölkörper eignen sich auch Polycarbonate, wie beispielsweise in WO 03/041676 beschrieben, auf die hier ausdrücklich Bezug genommen wird.

Als Polycarbonate besonders geeignet ist das unter der INCI Bezeichnung Hydrogenated Dimer Dilinoleyl / Dimethylcarbonate Copolymer, welches als Handelsprodukt Cosmedia^{®} DC von Cognis Deutschland GmbH & Co. KG erhältlich ist.
Dialkylether, Dialkylcarbonate, Triglycerid-Mischungen und Ester aus C8-C24-Fettsäuren und C8-C24 Fettalkoholen, Polycarbonate bzw. ein Gemisch dieser Substanzen sind erfindungsgemäß als Ölkörper besonders gut geeignet. Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen. Erfindungsgemäß bevorzugt ist es, ein Gemisch aus Ölkörpern einzusetzen, das Ester, Dialkylether und Triglyceride enthält.

Erfindungsgemäß einsetzbar sind u.a. auch Kohlenwasserstoffe, vorzugsweise mit einer Kettenlänge 8 bis 40 C-Atomen aus. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen ist, welche mehr als 1 Gew.-% 5,8-Diethyldodecan und/oder mehr als 1 Gew.-% Didecen enthalten.

In einer Ausführungsform der Erfindung, enthält die lipophile Phase mindestens ein Wachs.

Unter dem Begriff **Wachs** (synonym verwendet: **Wachskomponente**) werden üblicherweise alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

In einer bevorzugten Ausführungsform enthält die lipophile Phase mindestens ein **hydrophiles Wachs.** Hydrophile Wachse sind gekennzeichnet durch einen Schmelzpunkt von über 30°C und das Vorhandensein mindestens einer funktionellen OH-Gruppe. In Abgrenzung zu den Emulgatoren, weisen hydrophile Wachse einen HLB-Wert von kleiner 10 auf.

In einer bevorzugten Ausführungsform enthält die lipophile Phase als hydrophiles Wachs mindestens einen Partialester von C12 bis C 22 Fettsäuren mit mehrwertigen Alkoholen.

Die mehrwertige Alkohole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind:
- Glycerin
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- kurzkettige Alkyglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Typische Beispiele für geeignete Partialglyceride sind Mono- und/oder Diglyceride von C 12 bis C 22 Fettsäuren mit Glycerin sowie deren technische Gemische. Beispielsweise seien genannt langkettige Hydroxyfettsäure-monoglyceride, langkettige Hydroxyfettsäurediglyceride, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid.

Typische Beispiele für geeignete Partialgylceride sind Mono- und/oder Diglyceride von Dicarbonsäuren mit 4 bis 8 C-Atomen mit Glycerin sowie deren technische Gemische. Beispielsweise seien genannt Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozess noch geringe Mengen an Triglycerid enthalten können.
Ebenfalls geeignet sind Anlagerungsprodukte von 1 bis 30, vorzugsweise 5 bis 10 Mol Ethylenoxid an die genannten Partialglyceride.

Als hydrophile Wachse eingesetzt werden können Partialester von Glycerin und/oder Sorbitan -mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen, sofern sie einen Schmelzpunkt von > 30 °C aufweisen.

### Sorbitanester

Als Sorbitanester können beispielsweise die folgenden Verbindungen eingesetzt werden, sofern Sie einen Schmelzpunkt von > 30 °C aufweisen.

Als Sorbitanester kommen Sorbitanmonoisostearat, Sorbitansesquüsostearat, Sorbitandiisostearat, Sorbitantrüsostearat, Sorbitanmonooleat, Sorbitansesquioleat, Sorbitan-dioleat, Sorbitantrioleat, Sorbi-tanmonoerucat, Sorbitansesquierucat, Sorbitandierucat, Sorbitantrierucat, Sorbitanmonoricinoleat, Sor-bitansesquiricinoleat, Sorbitandiricinoleat, Sorbitantriricinoleat, Sorbitanmonohydroxystearat, Sorbitan-sesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantri-maleat sowie deren technische Gemische. Ebenfalls geeignet sind Anlagerungsprodukte von 1 bis 30, vorzugsweise 5 bis 10 Mol Ethylenoxid an die genannten Sorbitanester.

### Polyglycerinester

Als Polygylcerinester können beispielsweise die folgenden Verbindungen eingesetzt werden, sofern Sie einen Schmelzpunkt von > 30 °C aufweisen:

Typische Beispiele für geeignete Polyglycerinester sind Polyglyceryl-4 Düsostearate/Polyhydroxystearate/Sebacate (Isolan GPS), Polyglyceryl-2 Dipolyhydroxystearate (Dehy-muls PGPH), Polyglycerin-3-Diisostearate (Lameform TGI), Polyglyceryl-4 Isostearate (Isolan GI 34), Polyglyceryl-3 Oleate, Diisostearoyl Polyglyceryl-3 Diisostearate (Isolan PDI), Polyglyceryl-3 Methylglucose Distearate (Tego Care 450), Polyglyceryl-3 Beeswax (Cera Bellina), Polyglyceryl-4 Caprate (Polyglycerol Caprate T2010/90), Polyglyceryl-3 Cetyl Ether (Chimexane NL), Polyglyceryl-3 Distearate (Cremophor GS 32) und Polyglyceryl Polyricinoleate (Admul WOL 1403) Polyglyceryl Dimerate Isostearate sowie deren Gemische. Beispiele für weitere geeignete Polyolester sind die gegebenenfalls mit 1 bis 30 Mol Ethylenoxid um-gesetzten Mono-, Di- und Triester von Trimethylolpropan oder Pentaerythrit mit Laurinsäure, Kokosfett-säure, Talgfettsäure, Palmitinsäure, Stearinsäure, Ölsäure, Behensäure und dergleichen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter **Fetten** versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina^{®} HR im Handel ist. Ebenso geeignet sind Glycerintristearat, Glycerintribehenat (z. B. Syncrowax^{®} HRC), Glycerintripalmitat oder die unter der Bezeichnung Syncrowax^{®} HGLC bekannten TriglyceridGemische, mit der Vorgabe, daß der Schmelzpunkt der Wachskomponente bzw. des Gemisches bei 30 °C oder darüber liegt.

Als Wachs sind erfindungsgemäß insbesondere einsetzbar **Partialester** von C12 bis C22 Fettsäuren mit mehrwertigen Alkoholen, insbesondere mit Glycerin.

Als Wachs sind erfindungsgemäß insbesondere Mono- und Diglyceride bzw. Mischungen dieser **Partialglyceride** einsetzbar. Zu den erfindungsgemäß einsetzbaren Glyceridgemischen zählen die von der Cognis Deutschland GmbH & Co. KG vermarkteten Produkte Novata^{®} AB und Novata^{®} B (Gemisch aus C₁₂-C₁₈-Mono-, Di- und Triglyceriden) sowie Cutina^{®} MD oder Cutina^{®} GMS (Glycerylstearat).

Als Wachs einsetzbar sind weiterhin die **C₁₂-C₅₀-Fettalkohole.** Geeignet sind insbesondere C₁₂-C₂₄-Fettalkohole, die auch in Kombination mit den C12-C24 Partialestern von mehrwertigen Alkoholen einsetzbar sind. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole^{®}) oder die teilweise verzweigten Alkohole aus der Oxosynthese (Dobanole^{®}) verwendet werden. Erfindungsgemäß besonders bevorzugt geeignet sind C₁₄-C₂₂-Fettalkohole, die beispielsweise von der Cognis Deutschland GmbH unter der Bezeichnung Lanette^{®} 16 (C₁₆-Alkohol), Lanette^{®} 14 (C₁₄-Alkohol), Lanette^{®} O (C₁₆/C₁₈-Alkohol) und Lanette^{®} 22 (C₁₈/C₂₂-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C₁₄-C₄₀-**Fettsäuren** oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse**, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und **tierische Wachse**, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse**, wie z. B. Ceresin und Ozokerit oder die petrochemischen Wachse, wie z. B. Petrolatum, Paraffinwachse und Mikrowachse. Als Wachskomponente sind auch **chemisch modifizierte Wachse,** insbesondere die Hartwachse, wie z. B. Montanesterwachse, Sasolwachse und hydrierte Jojobawachse einsetzbar. Zu den **synthetischen Wachsen,** die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der **Wachsester** aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs^{®} K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetearylbehenat und Behenylbehenat einsetzbar.

In einer bevorzugten Ausführungsform der Erfindung enthält die lipophile Phase der Dispersionen mindestens einen Ölkörper ausgewählt aus der Gruppe bestehend aus Dialkylether, Dialkylcarbonate, Triglycerid-Mischungen, Ester aus C8-C24-Fettsäuren und C8-C24 Fettalkoholen, Polycarbonate bzw. ein Gemisch dieser Substanzen, Silikonöle und Mischungen hieraus.

In einer bevorzugten Ausführungsform der Erfindung ist das Gewichtsverhältnis von lipophiler Phase zu Emulgator größer 20:1, vorzugsweise größer 30:1, insbesondere größer 40:1., insbesondere größer 50:1.

Die erfindungsgemäßen Dispersionen ermöglichen es, große Mengen an lipophiler Phase im Vergleich zur eingesetzten Menge an Emulgator stabil zu dispergieren. Die so erhaltenen Dispersionen lassen sich verdünnen unter Erhalt der Tröpfchengrößenverteilung.
In einer Ausführungsform der Erfindung enthalten die Dispersionen
- 30 bis 55 Gew.-%, bevorzugt 40 bis 50 Gew.-% lipophile Phase
- Kleiner gleich 3 Gew.-% Emulgator, insbesondere kleiner gleich 2, bevorzugt kleiner gleich 1 Gew.-% Emulgator, wobei der Emulgator mindestens ein Acylglutamat enthält.

In einer bevorzugten Ausführungsform der Erfindung enthalten die Dispersionen
- 30 bis 55 Gew.-%, bevorzugt 40 bis 50 Gew.-% lipophile Phase
- Kleiner gleich 3 Gew.-% Emulgator, insbesondere kleiner gleich 2, bevorzugt kleiner gleich 1 Gew.-% Emulgator, wobei der Emulgator mehr als 30 Gew.-%, vorzugsweise mehr als 50 Gew.-% Acylglutamat enthält.

Die erfindungsgemäßen Dispersionen können **weitere Bestandteile** enthalten, wie beispielsweise Konservierungsmittel, biogene Wirkstoffe, UV-Lichtschutzfaktoren, Verdickungsmittel, Überfettungsmittel, Stabilisatoren, Polymere, Antioxidantien, Deodorantien, Filmbildner, Quellmittel, Insektenrepellentien, Hydrotrope, Solubilisatoren, Parfümöle, Farbstoffe etc. Die Mengen der jeweiligen Zusätze richten sich nach der beabsichtigten Verwendung.

In einer Ausführungsform der Erfindung enthaltend die erfindungsgemäßen Dispersionen als weiteren Bestandteil mindestens ein **Konservierungsmittel.**

Als **Konservierungsmittel** eignen sich beispielsweise Phenoxyethanol, Ethylhexylglycerin, Formaldehydlösung, Parabene, Pentandiol, Mischungen aus Phenoxyethanol und Ethylhexylglycerin (wie sie beispielsweise unter dem Handelsnamen Euxyl PE 9010 erhältlich sind) oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

In einer bevorzugten Ausführungsform der Erfindung wird das Konservierungsmittel ausgewählt aus der Gruppe bestehend aus Phenoxyethanol, Formaldehydlösung, Parabene, Pentandiol, Ethylhexylglycerin, organische Säuren und Mischungen daraus.

In einer Ausführungsform der Erfindung enthaltend die erfindungsgemäßen Dispersionen als weiteren Bestandteil mindestens ein **UV-Lichtschutzfilter.**

Als UV-Lichtschutzfilter geeignet sind bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben.

UV-B-Filter können öllöslich oder wasserlöslich sein. Als öllösliche Substanzen sind z.B. zu nennen:
- 3- Benzylidencampher bzw. 3-Benzylidennorcampher und dessen Derivate, z.B. 3-(4-Methylbenzy-liden)campher wie in der EP 0693471 B1 beschrieben
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino) benzoe-säureamylester;
- Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octo-crylene);
- Ester der Salicylsäure, vorzugsweise Salicylsäure-2-ethylhexylester, Salicylsäure-4-iso-propylben-zylester, Salicylsäurehomomenthylester;
- Derivate des Benzophenons, vorzugsweise 2-Hydroxy-4-methoxybenzophenon, 2-Hydroxy-4-me-thoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester
- Triazinderivate, wie z.B. 2,4,6-Trianilino-(p-carbo-2'-ethyl-1'-hexyloxy)-1,3,5-triazin und Octyl Tria-zon, wie in der EP 0818450 A1 beschrieben oder Dioctyl Butamido Triazone (Uvasorb HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der EP 0694521 B1 beschrieben.
- 2-(4-Diethylamino-2-hydroxybenzoyl)-benzoic acid Hexylester (Uvinul A plus)

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Erfindungsgemäß besonders günstige Kombinationen bestehen aus den Derivaten des Benzoylmethans, z.B. 4-tert.-Butyl-4'-methoxydibenzoyl-methan (Parsol 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepro-pylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Erfindungsgemäß bevorzugt sind UV-Lichtschutzfilter ausgewählt aus dem Anhang VII der europäischen Kosmetik-Gesetzgebung (24th Adapting Comission Directive, 29.Februar 2000)

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**) sowie** Parf.Kosm. 3, 11 (1999**)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distea-rylthiodi-pro-pionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoxi-min, Bu-tionin-sulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Man-nose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stil-benoxid, trans-Stil-benoxid) und die erfindungsgemäß geeigneten Derivate (Salze, Ester, Ether, Zucker, Nukleotide, Nukleoside, Peptide und Lipide) dieser genannten Wirkstoffe.

In einer Ausführungsform der Erfindung enthaltend die erfindungsgemäßen Dispersionen als weiteren Bestandteil mindestens einen **biogenen Wirkstoff.**

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

In einer bevorzugten Ausführungsform der Erfindung enthaltend die erfindungsgemäßen Dispersionen als biogenen Wirkstoff mindestens eine Verbindung ausgewählt aus Vitaminen, Allantoin, Bisabolol und Pflanzenextrakten.

In einer bevorzugten Ausführungsform der Erfindung enthaltend die erfindungsgemäßen Dispersionen als biogenen Wirkstoff mindestens eine Verbindung ausgewählt aus Tocopherol, Tocopherolacetat, Tocopherolpalmitat, Ascorbinsäure, β-Glucane, Retinol, Bisabolol, Allantoin, Phytantriol, Panthenol, AHA-Säuren, Pflanzenextrakten und Mischungen daraus.

In einer Ausführungsform der Erfindung enthaltend die erfindungsgemäßen Dispersionen als weiteren Bestandteil mindestens ein **Verdickungsmittel.**

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

In einer Ausführungsform der Erfindung enthaltend die erfindungsgemäßen Dispersionen als weiteren Bestandteil mindestens ein **desodorierenden Wirkstoff**.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Farbstoffe** können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.l.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage

Weitere Bestandteile der Dispersion (wie z.B. Konservierungsmittel, kosmetische Wirkstoffe, UV-Filter etc.) werden je nach ihrer Löslichkeit entweder über die Wasserphase oder über die lipophilen Phase zu der Dispersion gegeben.

### Herstellung der erfindungsgemäßen Dispersionen

Die erfindungsgemäßen Dispersionen werden durch Homogenisieren der nicht mischbaren Phasen hergestellt. Das Homogenisieren oder Dispergieren findet gegebenenfalls bei Temperaturen größer Raumtemperatur statt. In diesem Fall wird die Dispersion nach dem Dispergiervorgang wieder auf Raumtemperatur abgekühlt.

Unter Homogenisieren versteht man in der Dispergiertechnik das Feinstzerkleinern der dispersen Phase einer Rohemulsion. Dabei verschiebt sich im Fall der flüssig/flüssig Dispersion das Tröpfchengrößenspektrum der Rohemulsion deutlich hin zu kleineren Tropfen. Durch die Tropfenzerkleinerung entstehen neue Phasengrenzen, die durch Emulgatormoleküle schnell vollständig belegt werden müssen, da so der neu gebildete Tropfen besser stabilisiert wird und wegen der geringen Grenzflächenspannung leichter weiter zerkleinert werden kann.

Die erfindungsgemäßen Dispersionen können nach den üblichen Emulgiertechniken erhalten werden. Ein Gegenstand der Erfindung betrifft eine Verfahren zur Herstellung der erfindungsgemäßen Dispersionen, bei dem man die Wasserphase sowie die Mischung enthaltend Emulgator und die lipophile Phase vereinigt und die Homogenisierung mit einem Energie Eintrag von 1 x 10⁵ bis 2 x10⁸ J/m³ durchführt.

Als Homogenisierapparate können Hochdruckdispergiersysteme wie z.B. Radialdiffusoren mit Flachventil oder Zackenventil; Gegenstrahldispergatoren wie z.B. der Microfluidizer; Strahldispergatoren oder Blendensysteme eingesetzt werden. Als Dispergiersysteme eignen sich weiter Rotor-Stator-Systeme, Ultraschallsysteme, Kugelmühlen oder Membranen.

Beim Einsatz von Hochdruckdispergiersystemen als Homogenisierappart wird bei Drucken von 50 bis 2500 bar, vorzugsweise 200 bis 800 bar und insbesondere 400 bis 600 bar gearbeitet.

Bei einer Emulsionsherstellung mittels Micromischern ist ein Druckbereich von 2 bis 30, vorzugsweise von 5 bis 20 bar üblich. Mikromischer haben den Vorteil bei geringen Drucken besonders schonend feinteilige und enge Teilchengrößenverteilungen zu erzeugen

In einer bevorzugten Ausführungsform des erfindungsgemäßen Verfahrens wird die Homogenisierung mittels Hochdruckhomogenisierung durchgeführt.

Der Vorteil der Hochdruckhomogenisierung liegt darin, dass sehr leicht kleine Tröpfchen mit einer sehr engen Größenverteilung gebildet werden, was vorteilhaft ist, wenn niedrigviskose Dispersionen phasenstabil hergestellt werden sollen. Aufgrund der anwendungstechnischen Vorteile einer durch Hochdruckhomogenisierung hergestellten Emulsion wird zunehmend auch in der kosmetischen Industrie versucht derartige Homogenisiertechniken einzusetzen. Bedingt durch die Tatsache, dass eine neue Grenzfläche besonders schnell gebildet wird, sind hohe Anforderungen an Emulgator und Trägerphase zu stellen, da die Emulgatoren spontan und sehr schnell die Grenzfläche belegen müssen, um eine optimale Phasenstabilität sicher zu stellen.

Um feinteilige Dispersionen mit einer monomodalen und engen Teilchengrößenverteilung zu erzielen, insbesondere mit einem BTV Wert von kleiner gleich 2, kann es vorteilhaft sein, verschiedene Emulgierverfahren miteinander zu kombinieren. In einem Rührbehälter kann z.B. eine Vordispersion hergestellt werden, die dann durch Dispergierung in einer so genannten Einmalpassage mittels eines Rotor-Statur-Systems und anschließend durch einen Hochdruckhomogenisator homogenisiert werden. Unter Einmalpassage ist hier eine Verfahrensweise zu verstehen, in welcher der gesamte Inhalt eines Behälters einmal durch den Homogenisierapparat in einen anderen Behälter gefahren wird. Im Gegensatz zur sog. Kreislauffahrweise ist so gewährleistet, dass jedes Flüssigkeitselement einmal den Homogenisierapparat passiert hat. Es bleiben keine groben Emulsionströpfchen zurück, die den Ausgangspunkt für den Zerfall der Dispersion bilden können.

Zum Vordispergieren werden vorzugsweise Rotor-Stator-Systeme eingesetzt. Rotor-Stator-Systeme können Apparate sein wie Zahnkolloidmühlen oder Maschinen die aus einen oder mehreren Rotoren und Statoren mit Durchtrittsöffnungen in Form von Schlitzen oder zylindrischen oder rechteckigen Löchern bestehen, wie z.B. vom Typ Cavitron, Supraton, Siefer, Bran+Lübbe, IKA, Koruma, Silverson usw.

In einer bevorzugten Ausführungsform der Erfindung werden der oder die Homogenisierungsschritte mehrfach durchlaufen.

Die erfindungsgemäßen Dispersionen können in einer Ausführungsform der Erfindung durch Herstellung von Konzentraten und anschließendes Verdünnen mit Wasser hergestellt werden. Dies kann insbesondere bei Dispersionen mit geringer Endkonzentration an lipophiler Phase von Vorteil sein.

### Gewerbliche Anwendbarkeit

Die erfindungsgemäßen Dispersionen eignen sich zur Verwendung in kosmetischen und/oder pharmazeutischen Zubereitungen. Beispiele sind sprühbare Dispersionen für die Körperpflege (Deosprays, Sonnenschutzsprays usw.), Pflegeprodukte in Gel- oder Cremeform, arzneimittelhaltige Sprays, Gele oder Cremes, getränkte Pflegetücher oder Pads (Make-up-Entferner, Reinigungstücher usw.) und Ähnliches.

Ein weiterer Gegenstand der Erfindung betrifft daher die Verwendung der erfindungsgemäßen Dispersionen in kosmetischen und/oder pharmazeutischen Zubereitungen, insbesondere zur Herstellung von kosmetischen und/oder pharmazeutischen Zubereitungen.

Die Dispersionen sind besonders geeignet zur Applikation auf Papieren, Tüchern, Textilien und Watteprodukten, die im Bereich der Babypflege und -hygiene sowie im Bereich der Make-up-Entfernung, insbesondere der Augen-Make-up-Entfernung, im Bereich der Damenhygiene (Tampons, Damenbinden, Slip-Einlagen) und im Bereich der Körperhygiene (Toilettenpapier, Feuchttoilettenpapier) eingesetzt werden.

Marktübliche Reinigungstücher werden entweder mit wässrigen Lotionen basierend auf ethoxylathaltigen (EO) Emulgatoren, wie z. B. in WO 00/04230 beschrieben oder aber mit wässrigen, klaren Solubilisaten getränkt. Die erstgenannten wässrigen Lotionen werden aufgrund des EO-Gehaltes in verschiedenen Märkten nicht akzeptiert (Öko-Konform). Die klaren Solubilisate sind dagegen häufig sehr klebrig und sensorisch nicht akzeptabel. Da von Solubilisaten aufgrund der Klebrigkeit oft sehr starke Verdünnungen eingesetzt werden müssen, besitzen die damit produzierten Tücher nur noch eine geringe Reinigungsleistung. Die erfindungsgemäßen Dispersionen enthalten geringe Emulgator- und hohe Öl-und Wachsmengen. Daher haben Sie eine sehr hohe Reinigungsleistung. Zudem enthalten die Dispersionen in einer bevorzugten Ausführungsform keine ethoxylierten Emulgatoren.

Ein weiter Gegenstand der Anmeldung ist daher die Verwendung der erfindungsgemäßen Dispersionen auf Papieren, Vliese (Nonwoven) und Geweben (Woven), insbesondere die Verwendung zur Applikation auf oder Ausrüstung von Papieren, Vliese (Nonwoven) und Geweben (Woven). Erfindungsgemäß zählen hierzu alle Papierarten, Vliese und Gewebe, die dem Fachmann geläufig sind, und Produkte, die daraus herstellbar sind, wie z.B. Toilettenpapier, Papiertaschentücher, Tissues, Wipes, Watte, Wattepads, Make-up Entferner, Tampons, Binden, Slip-Einlagen, Windeln, Babypflegetücher, Babyreinigungstücher, Textilien, etc. Ebenso Gegenstand der Anmeldung sind Papier-, Vlies- und Gewebe-Produkte zur Körperpflege und -reinigung, welche eine erfindungsgemäße Dispersion enthalten.

Als Substrate dieser Papier-, Vlies- und Gewebe-Produkte seinen exemplarisch genannt: Träger aus Textilfaser, z.B. aus Naturfaser, wie Cellulose, Seide, Wolle, Regeneratcellulose (Viskose, Rayon), Cellulosederivate und/oder synthetische Fasern, wie z.B.Polyester-, Polypropylen-, Polyethylenterephtalat-, Polyamin-, Polyolifin-, Polyacrylnitril-Fasern oder Mischungen solcher Fasern, gewebt oder ungewebt.

Die erfindungsgemäßen Produkte können nach dem Fachmann bekannten Verfahren hergestellt werden. Der Auftrag der erfindungsgemäßen Dispersionen auf die erfindungsgemäßen Papier-, Vlies- und Gewebe-Produkte zur Körperpflege und -reinigung erfolgt dabei nach dem Fachmann bekannten Methoden, wie beispielsweise Imprägnieren, Tränken, Eintauchen, Ansprühen, Abstreifen oder Beschichten. Dies kann sowohl bei Raumtemperatur als auch bei erhöhten Temperaturen erfolgen.

Die erfindungsgemäße Dispersion kann vor dem Auftrag auf das Papier-, Vlies- und Gewebe-Substrat verdünnt werden und gegebenenfalls kann das erhaltene Papier-, Vlies- und Gewebe-Produkt anschließend getrocknet werden.

Ein Gegenstand der Erfindung betrifft ein Papier-, Vlies- und/oder Gewebe-Produkte zur Körperpflege und/oder zur Körperreinigung, welches 0,5 bis 70 Gew.-% an Aktivsubstanz der Dispersion enthält, bezogen auf das Gesamtgewicht des Produktes.

In einer bevorzugten Ausführungsform der Erfindung enthalten die Papier-, Vlies- und/oder Gewebe-Produkte mehr als 2, insbesondere mehr als 3, besonders bevorzugt mehr als 4, insbesondere mehr als 5 Gew.-% an Aktivsubstanz der Dispersion bezogen auf das Gesamtgewicht des Produktes.

In einer bevorzugten Ausführungsform der Erfindung enthalten die Papier-, Vlies- und/oder Gewebe-Produkte 10 bis 60, insbesondere 15 bis 50, bevorzugt 20 bis 45 Gew.-% an Aktivsubstanz der Dispersion bezogen auf das Gesamtgewicht des Produktes.

Als **Aktivsubstanz** der Dispersion werden alle Bestandteile der Dispersion abzüglich des Wassers verstanden.

Die Papier-, Vlies- oder Gewebe-Produkte können in einem Trocknungsschritt nachbehandelt werden, um den Wassergehalt nach der Sprüh-Applikation zu reduzieren oder um nahezu wasserfreie Produkte zu erhalten (z.B. Dry-Wipes). In einer Ausführungsform der Erfindung werden erfindungsgemäß beschichteten Papier-, Vlies- und Gewebe-Produkte nachfolgend einem Trocknungsschritt unterworfen werden.

Ein weiterer Gegenstand der Erfindung betrifft die Verwendung der erfindungsgemäßen Dispersionen auf Papieren, Vliesen und/oder Geweben, insbesondere als Imprägnier- und/oder Avivagemittel.

Weiterhin wurde überraschenderweise festgestellt, dass Dispersionen, enthaltend Phospholipide, insbesondere Lecithine, das Pflegegefühl auf der Haut, welche mit dem Papier-, Vliess- und Gewebeprodukten in Kontakt kommt, steigert.

Weiterhin wurde überraschenderweise festgestellt, dass Dispersionen, enthaltend Phospholipide, insbesondere Lecithine, den Weichgriff des Papier-, Vliess- und Gewebeprodukten steigert.

### Beispiele

### Herstellung der Dispersion (Emulsion) nach Tabelle 1, Beispiel 12

20 g Cetiol™SN (INCI: Cetearyl Isononanoate), 5 g Cutina™PES (INCI: Pentaerythrityl Disterarate), 3 g Glycerin und 2 g Leciprime™1800 IP (INCI: Lecithin, Hersteller Fa. Cargill) wurden bei 60 °C in einem Becherglas unter Rühren gelöst bzw. vordispergiert. In einem zweiten Becherglas wurde 1 g Acylglutamat (Eumulgin™SC) in 60 °C warmem destilliertem Wasser gelöst. Zu dieser wässrigen Phase wurde das Konservierungsmittel Uniphen P-23 (Hersteller Fa. Induchem) gegeben. Die Ölphase wurde unter Rühren in die wässrige Phase gegeben und mit Hilfe eines Ultra Turrax (fa. IKA Typ T50, Werkzeug S50N-G40G) bei 5200 U/min für 2 Minuten vorhomogenisiert. Anschließend wurde die Voremulsion in einem weiteren Homogenisierungsschritt mit einem Hochdruckhomogenisator (Fa. APV, LAB 60) bei 750 bar homogenisiert. Dieser Homogenisierungsschritt wurde insgesamt 5x durchlaufen. Tabelle 1 zeigt erfindungsgemäße Dispersionen (Emulsionen), Beispiele 1 bis 20.

### Lagerversuche

Zur Untersuchung der Lagerstabilität (Phasenstabilität) der erfindungsgemäßen Dispersionen wurden diese bei Raumtemperatur (23 °C) und bei 45 °C 6 Wochen gelagert. Ebenso wurden verdünnten Dispersionen hergestellt, indem die Dispersionen mit Wasser auf einen Dispersionensgehalt von 10 Gew.-% verdünnt wurden.

Die Tropfen-/Partikelgrößenverteilung der unverdünnten sowie der verdünnten Dispersionen wurde vor, währen und nach den Lagerversuchen bestimmt, die erhaltenen d₉₀ Wert und das Aussehen der Dispersionen wurde zur Beurteilung der Lagerstabilität herangezogen.
Der so erhaltene d Wert (genauer d_{3,90}-Wert) beschreibt, dass 90% des Volumens der dispersen (emulgierten) Phase von Tropfen mit einem Durchmesser < = d_{3,90} gebildet wird.
Die Teilchengrößenverteilung wurde mit einem Gerät der Fa. Beckmann Coulter, Typ LS 230, unter Nutzung des optischen Models Emulsiond.rfd PIDS included (vom 14.8.2001) nach Bedienungsanleitung (1994) bestimmt. Als Messmedium wurde Wasser verwendet. Die Messung der Teilchengröße erfolgte unmittelbar nach der Herstellung der Dispersionen bzw. im Verlauf der Lagerversuche. Nach den Vorgaben des Geräteherstellers wurden jeweils verdünnte Dispersionen gemessen, d.h. es wurde soviel der Dispersionen in destilliertes Wasser eingerührt bis die gerätespezifische Sättigungskonzentration vom Gerät angezeigt wurde. Tabelle 2 zeigt die Ergebnisse für diese Lagerversuche.
1) Alle Beispiele wurden mit dem Hochdruckhomogenisator APV, LAB 60 durchgeführt, die Homogenisation wurde 5x durchgeführt, in Beispiel 5 wurde die Homogenisation nur 2x durchgeführt.

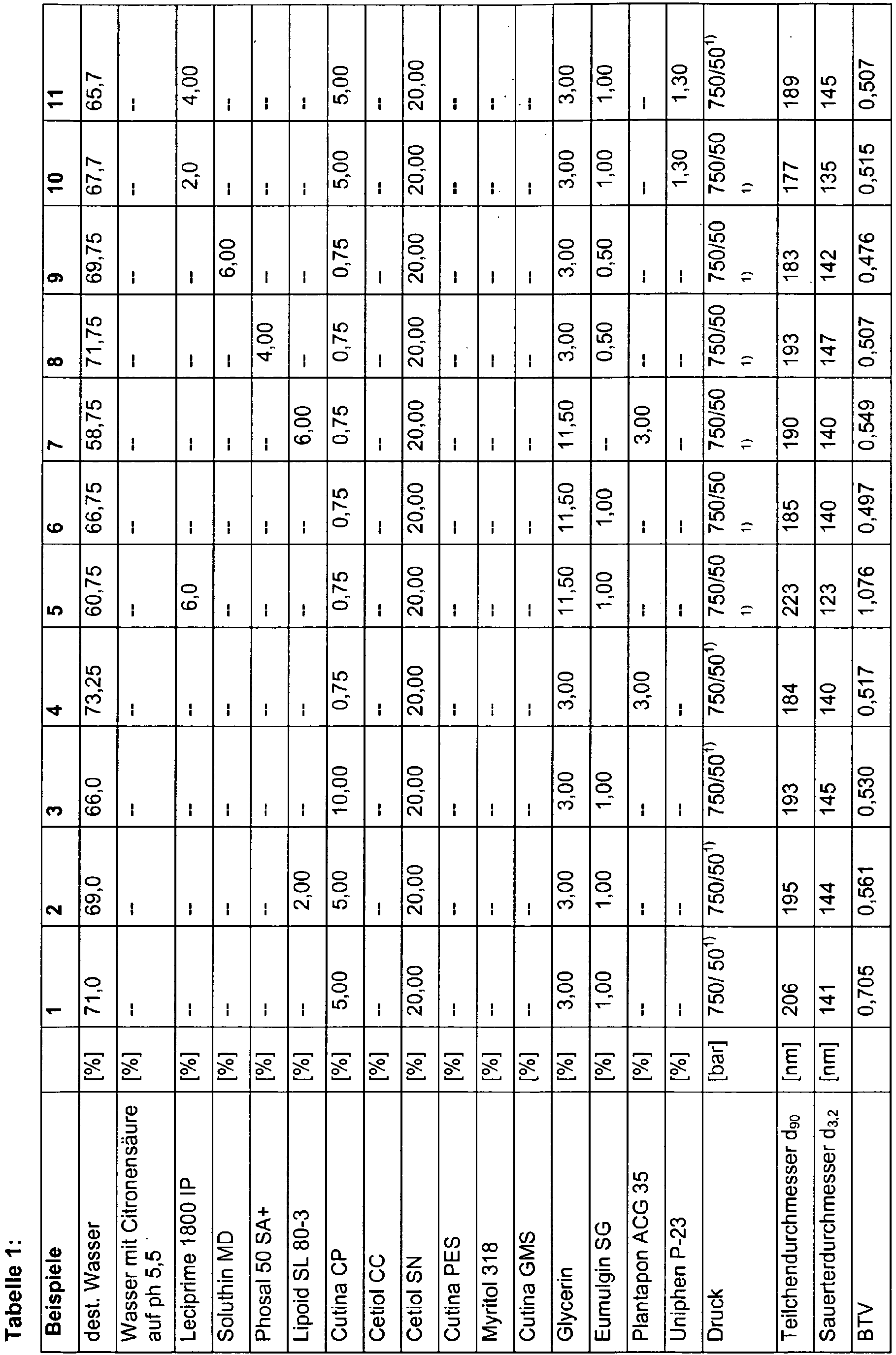

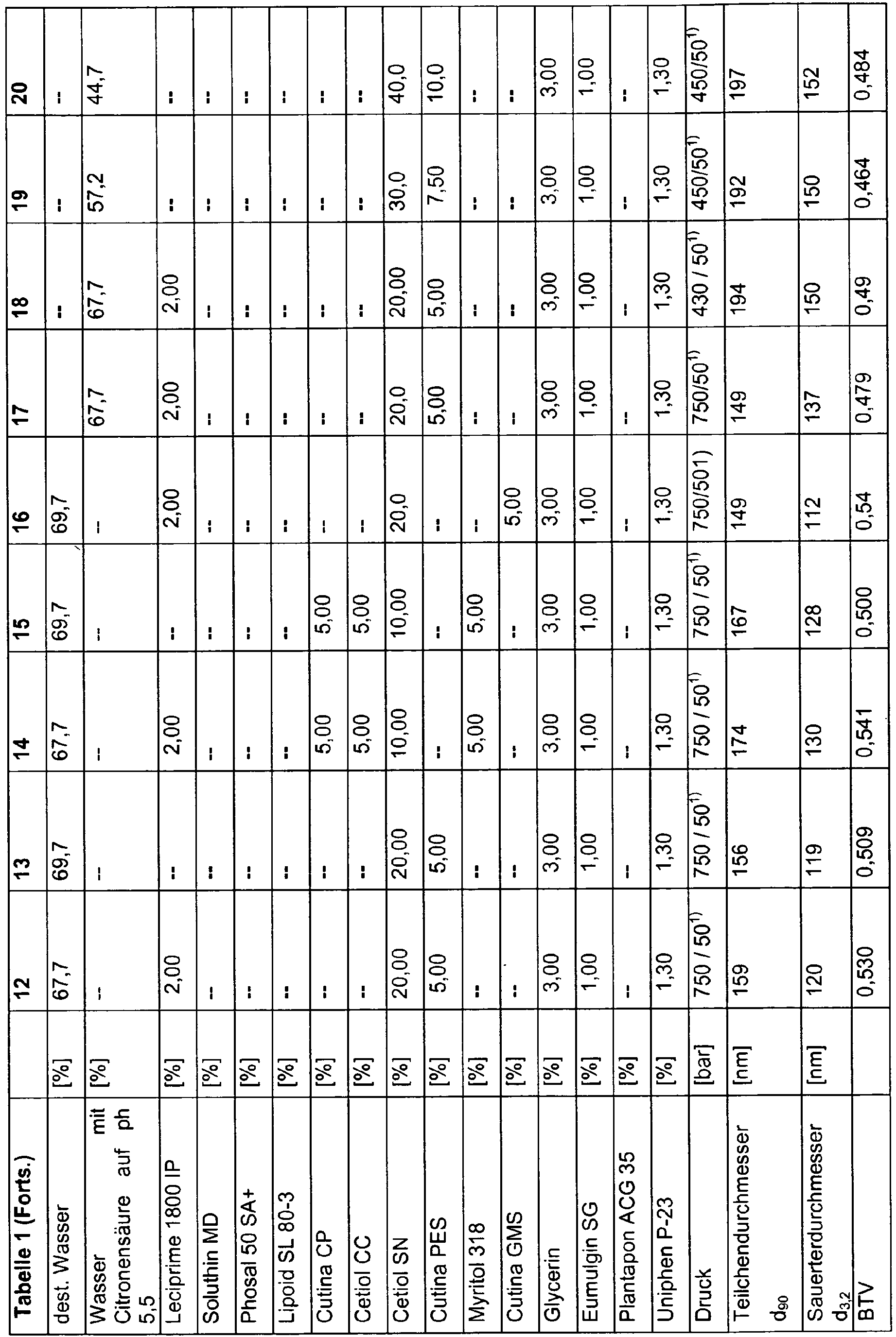

**Tabelle 2: Lagerversuche ausgewählter Rezepturen**

| Beispiel Nr. | | 5 | 6 | 8 | 10 | 12 | 13 | 14 | 15 | 16 |
|---|---|---|---|---|---|---|---|---|---|---|
| | | | | | | | | | | |
| d₉₀ original Emulsion Start | [nm] | 223 | 185 | 199 | 184 | 163 | 158 | 181 | 172 | 157 |
| d₉₀ verdünnte Emulsion Start | [nm] | 223 | 184 | 198 | 184 | 162 | 159 | 181 | 172 | 156 |
| d₉₀ original Emulsion 6 Wochen bei RT | [nm] | 224 | 185 | 198 | 189 | 166 | 166 | 184 | 177 | 160 |
| d₉₀ original Emulsion 6 Wochen bei 45°C | [nm] | 224 | 189 | 187 | 190 | 168 | 168 | 186 | 181 | 159 |
| d₉₀ verdünnte Emulsion 6 Wochen bei RT | [nm] | 223 | 185 | 198 | 190 | 166 | 165 | 187 | 176 | 159 |
| d₉₀ verdünnte Emulsion 6 Wochen bei 45°C | [nm] | 223 | 187 | 186 | 190 | 166 | 167 | 189 | 179 | 159 |

| Aussehen | | | | | | | | | | |
|---|---|---|---|---|---|---|---|---|---|---|
| d₉₀ original Emulsion Start | | dünnflüssig, keineTrennung | | | | | | | | |
| d₉₀ verdünnte Emulsion Start | | dünnflüssig, keineTrennung | | | | | | | | |
| d₉₀ original Emulsion 6 Wochen bei RT | | dünnflüssig, keineTrennung | | | | | | | | |
| d₉₀ verdünnte Emulsion 6 Wochen bei RT | | dünnflüssig, keineTrennung | | | | | | | | |
| d₉₀ original Emulsion 6 Wochen bei 45°C | | dünnflüssig, keineTrennung | | | | | | | | |
| d₉₀ verdünnte Emulsion 6 Wochen bei 45°C | | dünnflüssig, keineTrennung | | | | | | | | |

### Sensorische Bewertung

Zur Bewertung der sensorischen Eigenschaften der erfindungsgemäßen Emulsionen, wurden von Beispiel 12 und 13 (siehe Tabelle 1) 20 Gew.-%ige Verdünnungen mit Wasser hergestellt. Von diesen Verdünnungen wurden jeweils 5 g auf Tücher (Spunlace, 180mm x 200 mm, 55 gsm, 65% Viskose Rayon/ 35 % Polyester) aufgetragen und von einem Expertenpanell bestehend aus 12 Personen sensorisch nach den folgenden Kriterien bewertet:
- Weichheit 1 = Beurteilung, wie weich oder rau sich das Tuch insgesamt in der Hand anfühlt
- Weichheit 2 = Beurteilung wie wich oder rau sich das Tuch bei Berührung mit dem Unterarm anfühlt
- Klebrigkeit
- Stumpfheit
- Pflegegefühl

Die Bewertung erfolgt anhand von Noten von 1 bis 7, wobei die Note 1 "wenig", die Note 7 "viel" bedeutet.

Beispiele 12 und 13 aus Tabelle 1 wurden so bewertet:

| Kriterium | Beispiel 12 | Beispiel 13 |
|---|---|---|
| Weichheit 1 | 7 | 5 |
| Weichheit 2 | 7 | 5 |
| Klebrigkeit | 2 | 3 |
| Stumpfheit | 1 | 3 |
| Pflegegefühl | 7 | 5 |

Die Beispiele zeigen, dass die bereits mit den der erfindungsgemäßen Dispersion erhaltenen guten sensorischen Eigenschaften (Beispiel 13) durch den Zusatz von Lecithin (Beispiel 12) noch verbessert werden können.

## Patentansprüche

1. Dispersion enthaltend Wasser, lipophile Phase und Emulgator, **dadurch gekennzeichnet, dass** die Dispersion weiterhin mindestens ein Phospholipid enthält, dass der Emulgator in einer Menge von kleiner gleich 3 Gew.-% bezogen auf das Gesamtgewicht der Dispersion zugegen ist, dass der Emulgator mindestens ein Acylglutamat enthält und dass der Emulgator weniger als 10 Gew. % ethoxylierte Emulgatoren enthält.

2. Dispersion nach Anspruch 1, **dadurch gekennzeichnet, dass** der Emulgator mehr als 30 Gew. %, vorzugsweise mehr als 50 Gew.-% Acylglutamate enthält.

3. Dispersion nach mindestens einem der vorgenannten Ansprüche, **dadurch gekennzeichnet, dass** der Emulgator weniger als 5 Gew. % ethoxylierte Emulgatoren enthält.

4. Dispersion nach mindestens einem der vorgenannten Ansprüche, **dadurch gekennzeichnet, dass** der Sauter-Durchmesser D[3,2], kleiner gleich 400 nm, insbesondere kleiner gleich 200 nm beträgt.

5. Dispersion nach mindestens einem der vorgenannten Ansprüche, **dadurch gekennzeichnet, dass** sie als Phospholipid mindestens ein Lecithin enthält.

6. Verfahren zur Herstellung von Dispersionen nach Anspruch 1, bei dem man die Wasserphase sowie die Mischung enthaltend Emulgator und die lipophile Phase vereinigt und die Homogenisierung mit einem Energie Eintrag von 1×10⁵ bis 2×10⁸ J/m³ durchführt.

7. Verwendung einer Dispersion nach mindestens einem der vorgenannten Ansprüche in kosmetischen und/oder pharmazeutischen Zubereitungen.

8. Verwendung einer Dispersion nach mindestens einem der vorgenannten Ansprüche auf Papieren, Vliesen und/oder Geweben.

9. Papier-, Vlies- und Gewebe-Produkte zur Körperpflege und/oder zur Körperreinigung, die eine Dispersion nach mindestens einem der vorgenannten Ansprüche enthält.

## Claims

1. Dispersion comprising water, lipophilic phase and emulsifier, **characterized in that** the dispersion further comprises at least one phospholipid, **in that** the emulsifier is present in an amount of less than or equal to 3% by weight based on the total weight of the dispersion, **in that** the emulsifier comprises at least one acylglutamate, and **in that** the emulsifier comprises less than 10% by weight of ethoxylated emulsifiers.

2. Dispersion according to Claim 1, **characterized in that** the emulsifier comprises more than 30% by weight, preferably more than 50% by weight, of acylglutamates.

3. Dispersion according to at least one of the preceding claims, **characterized in that** the emulsifier comprises less than 5% by weight of ethoxylated emulsifiers.

4. Dispersion according to at least one of the preceding claims, **characterized in that** the Sauter diameter D[3,2], is less than or equal to 400 nm, in particular less than or equal to 200 nm.

5. Dispersion according to at least one of the preceding claims, **characterized in that** it comprises at least one lecithin as phospholipid.

6. Process for the preparation of dispersions according to Claim 1, in which the water phase and also the mixture comprising emulsifier and the lipophilic phase are combined and the homogenization is carried out with an energy input of from 1 × 10⁵ to 2 × 10⁸ J/m³.

7. Use of a dispersion according to at least one of the preceding claims in cosmetic and/or pharmaceutical preparations.

8. Use of a dispersion according to at least one of the preceding claims on papers, nonwovens and/or wovens.

9. Paper, nonwoven and woven products for bodycare and/or for cleaning the body, which comprise a dispersion according to at least one of the preceding claims.

## Revendications

1. Dispersion contenant de l'eau, une phase lipophile et un émulsifiant, **caractérisée en ce que** la dispersion contient en outre au moins un phospholipide, **en ce que** l'émulsifiant est présent en une quantité inférieure à 3% en poids par rapport au poids total de la dispersion, **en ce que** l'émulsifiant contient au moins un glutamate d'acyle et **en ce que** l'émulsifiant contient moins de 10% en poids d'émulsifiants éthoxylés.

2. Dispersion selon la revendication 1, **caractérisée en ce que** l'émulsifiant contient plus de 30% en poids, de préférence plus de 50% en poids, de glutamates d'acyle.

3. Dispersion selon au moins quelconque l'une des revendications susmentionnées, **caractérisée en ce que** l'émulsifiant contient moins de 5% en poids d'émulsifiants éthoxylés.

4. Dispersion selon au moins quelconque l'une des revendications susmentionnées, **caractérisée en ce que** le diamètre de Sauter D[3,2] est inférieur à 400 nm, en particulier inférieur à 200 nm.

5. Dispersion selon au moins quelconque l'une des revendications susmentionnées, **caractérisée en ce qu'**elle contient, comme phospholipide, au moins une lécithine.

6. Procédé pour la préparation de dispersions selon la revendication 1, dans lequel on réunit la phase aqueuse ainsi que le mélange contenant l'émulsifiant et la phase lipophile et on réalise l'homogénéisation avec une introduction d'énergie de 1 x 10⁵ à 2 x 10⁸ J/m³.

7. Utilisation d'une dispersion selon au moins l'une quelconque des revendications susmentionnées dans des compositions cosmétiques et/ou pharmaceutiques.

8. Utilisation d'une dispersion selon au moins l'une quelconque des revendications susmentionnées sur des papiers, des non-tissés et/ou des tissus.

9. Produits en papier, en non-tissé et en tissu pour les soins corporels et/ou le nettoyage corporel, qui contiennent une dispersion selon au moins l'une quelconque des revendications susmentionnées.
